# EUROPEAN PATENT SPECIFICATION

(11) **EP 2 459 745 B1**
(45) Date of publication and mention of the grant of the patent: **12.11.2014**
(21) Application number: 10740163.0
(22) Date of filing: 29.07.2010
(51) Int. Cl.: C12Q 1/68

(54) **IMPROVED DETECTION OF BACTERIAL (MOLLICUTES) CONTAMINATION**
VERBESSERTER NACHWEIS VON BAKTERIELLER (MOLLICUTES) KONTAMINATION
DÉTECTION AMÉLIORÉE DE CONTAMINATION BACTÉRIENNE (MOLLICUTES)

(30) Priority: 01.08.2009 EP 09009965
(43) Date of publication of application: 06.06.2012
(73) Proprietor: Roche Diagnostics GmbH, 68305 Mannheim (DE); F.Hoffmann-La Roche AG, 4070 Basel (CH)
(72) Inventor: BIRKNER, Christian, 82449 Uffing (DE)
(74) Representative: Burger, Alexander
(86) International application number: PCT/EP2010/004655
(87) International publication number: WO 2011/015312

(56) References cited:
- ELDERING J A ET AL: "Development of a PCR method for mycoplasma testing of Chinese hamster ovary cell cultures used in the manufacture of recombinant therapeutic proteins", BIOLOGICALS, ACADEMIC PRESS LTD., LONDON, GB, vol. 32, no. 4, 1 December 2004 (2004-12-01), pages 183-193, XP004657306, ISSN: 1045-1056

## Description

The present invention provides an improved PCR-based amplification of a target sequence by suppressing non-specific amplification products. The improvement concerns the use of a primer pair optimized to amplify a nucleic acid of a contaminant in the background of genomic DNA of a first organism. When DNA from a second organism suspected for comprising the contaminant is subjected to the same PCR-based amplification reaction, detection sensitivity and specificity of the contaminant is enhanced when an amount of genomic DNA of the first organism is present in the amplification reaction.

### Background of the Invention

Mycoplasma is a genus of bacteria belonging to the class of Mollicutes which lack a cell wall. Without a cell wall, mycoplasma bacteria are unaffected by many common antibiotics such as penicillin or other beta-lactam antibiotics that target prokaryotic cell wall synthesis. There are over 100 recognized species of the genus Mycoplasma, one of several genera in the Mollicutes. Mollicutes are parasites or commensals of humans, other animals (including insects), and plants; the genus Mycoplasma is by definition restricted to vertebrate hosts. Cholesterol is required for the growth of species of the genus Mycoplasma as well as certain other genera of mollicutes. Their optimum growth temperature is often the temperature of their host if warmbodied (e.g. 37°C in humans) or ambient temperature if the host is unable to regulate its own internal temperature. Analysis of 16S ribosomal RNA sequences as well as gene content strongly suggest that the mollicutes, including the mycoplasmas, are closely related to either the Lactobacillus or the Clostridium branch of the phylogenetic tree (Firmicutes *sensu stricto*).

Mycoplasma species are often found in research laboratories as contaminants in cell culture. Mycoplasmal cell culture contamination can occur due to contamination from individuals or contaminated cell culture medium ingredients. Mycoplasma cells are physically small - less than 1 µm - and they are therefore difficult to detect with a conventional microscope. Mycoplasmas may induce cellular changes, including chromosome aberrations, changes in metabolism and cell growth. Severe Mycoplasma infections have the potential to destroy a cell line.

Mycoplasmas are also involved as pathogens in a number of diseases. Mycoplasma pneumoniae is the major causative agent of community-acquired pneumonia. Spiroplasma species have been linked recently to the transmissible spongiform encephalopathies (TSEs), by molecular and serological studies (Bastian, F.O., J Neuropathol Exp Neurol 64 (2005) 833-838). However, an association of Spiroplasma species with TSEs is still controversial, e.g. in view of the failure of a blinded study of rRNA species to detect any footprint of Spiroplasma in scrapie-infected hamster brain (Alexeeva, I., et al., J Clin Microbiol 44 (2006) 91-97). Nevertheless, the TSEs under consideration include scrapie in sheep, chronic wasting disease (CWD) in deer and Creutzfeldt-Jakob disease in humans. Spiroplasma spp. isolates from scrapie-affected sheep brain and from CWD-affected deer brain inoculated intra-cranially into sheep and goats induced spongiform encephalopathy closely resembling natural TSE in these animals. These data apparently show spiroplasma to be associated with TSE.

It was shown that Spiroplasma bacteria grow in embryonated eggs and can be passaged in such a culture system (Bastian, F.O., et al., Journal of Medical Microbiology 56 (2007) 1235-1242).

Embryonated eggs play a major role in the production of vaccines. For example, human vaccines against influenza have been available for almost 60 years and, until recently, were prepared almost entirely from viruses grown in the allantoic cavity of 9- to 11-day-old embryonated chicken eggs. Thus, a pathogenic potential of Spiroplasma makes detection of a Mollicutes pathogen a tool of primary importance for securing the safety of any product prepared from embryonated eggs.

Moreover, the possibility of Mycoplasma contamination during biopharmaceutical production, cell therapy, and tissue engineering is a major problem in particular. Traditional detection methods, which are required by Pharmacopoeias and drug regulators worldwide, use growth on culture media to identify contaminating organisms. These culture-based techniques require long time to achieve results. Furthermore, cultivation of certain Mycoplasma species may not be possible or reliable. Therefore, improved and particularly more rapid and reliable microbial assays are needed.

Eldering, J.A., et al., Biologicals 32 (2004) 183-193 disclose a PCR method for mycoplasma testing of Chinese hamster ovary cell cultures. The principle of the test is to firstly isolate genomic DNA from a cell culture (cells and supernatant), and secondly to perform a polymerase chain reaction (PCR) using primers specific for a region in the 16S-rRNA gene of mycoplasma species and the isolated DNA as a template. In case of a positive result an amplification product corresponding to the targeted region is formed and detected. A negative result is obtained when no amplification product is formed.

The primers used in the method are universal mycoplasma primers which were published previously (Wong-Lee, J.G. and Lovett, M., "Rapid and sensitive PCR method for identification of Mycoplasma species in tissue culture" in: Diagnostic molecular microbiology - principles and applications; Persing, DH, Smith, TF, Tenover, FC, White, TJ (editors), Washington, DC, American Society for Microbiology (1993) 257-260). The method of Eldering, J.A., et al. *(supra)* is the result of assay optimization and combines six elements which improve sensitivity and specificity of mycoplasma detection: (1) a positive control plasmid for a PCR product with a distinct size compared to amplificates of a mycoplasma genome, (2) purification and concentration of the DNA isolated from the cell cultures suspected of being contaminated with mycoplasma bacteria, (3) use of a hot-start *Taq* DNA polymerase, (4) use of a so-called touch-down PCR technique in which an annealing temperature gradient from 70 to 60°C is applied, (5) decontamination of PCR reagents, and (6) use of dedicated equipment and materials.

Notably, concerning element (2) of said optimized PCR method the authors selected a method for DNA purification and concentration comprising the steps of (a) lysing the cell culture (cells and medium), and (b) precipitating DNA from the lysate with ethanol and isolating the precipitated DNA.

It is further disclosed that such a precipitation method is advantageous over an alternative method for DNA purification using spin columns. Spin columns typically contain a filter material with a silica surface. DNA dissolved in a high-salt and/or chaotropic buffer which optionally also contains an alcohol is bound to the filter material by passing the buffer through the spin column, e.g. by way of centrifugation. In a subsequent step DNA can be eluted from the filter material using a non-chaotropic low-salt buffer or pure water. In the optimization study results were obtained which are consistent with a trace contaminant which might be present in the spin colums. When the columns were contacted with CHO genomic DNA traces of the contaminant were removed from the spin columns. The probable effect of the CHO genomic DNA was described as that of a carrier for the contaminant.

Based on the findings of Eldering, J.A., et al. *(supra)* and the workflow disclosed therein Roche Applied Science (Roche Diagnostics GmbH, Mannheim, Germany) has further optimized and developed the MYCOTOOL test kit. The assay performed using the test kit provides a highly sensitive Mycoplasma assay for both cellular and cell free systems. MYCOTOOL is particularly directed to pharmaceutical quality control.

Previous studies were directed to PCR-generated artefacts from 16S rRNA gene-specific primers (Osborne, C.A., et al., FEMS Microbiology Letters 248 (2005) 183-187). It was concluded that artefacts generated using a certain pair of primers can be avoided by using different primers for the amplification reaction.

The inventor of the present invention has found occasional cases where the PCR of MYCOTOOL assay produced fragments which could be artifacts (see Examples). Having in mind the fact that the optimitzed assay of Eldering, J.A., et al. *(supra)* and particularly the primers used therein basically constitute a very good detection system for mollicutes, it was the object of the invention to improve the PCR process such that the occurrence of irregular amplificates is minimized. Particularly this desired effect was to be achieved without changing the sequence of the primers.

### Summary of the Invention

According to the invention, one or more of the above-mentioned objectives were met by the embodiments of the present invention.

A first aspect of the invention is an improved method for determining the presence or absence of a bacterial contaminant in a liquid sample with biological material, the bacterial contaminant being selected from the group consisting of Mycoplasma hyorhinis, Mycoplasma arginini, Mycoplasma pneumoniae, Mycoplasma fermentans, Mycoplasma orale, Mycoplasma pirium, Acholeplasma laidlawii and Spiroplasma mirium, and said method comprising the steps of (a) processing the sample and purifying the nucleic acids from the processed sample; followed by (b) forming a composition for a PCR-based amplification reaction (reaction mixture), the composition including a first primer according to SEQ ID NO:1, a second primer according to SEQ ID NO:2, and the purified nucleic acids of step (a) or a measured fraction thereof as a template; followed by (c) performing a polymerase chain reaction (PCR) with the composition of step (b); followed by (d) detecting the presence or absence of an amplified target sequence, wherein the presence of said amplified target sequence indicates the presence of the bacterial contaminant in the sample, and the absence of said amplified target sequence indicates the absence of the bacterial contaminant in the sample; the improvement being characterized in that relative to the volume of the sample a predetermined amount of DNA from CHO cells is added to (i) the sample, or (ii) the processed sample of step (a), or (iii) the purified nucleic acids obtained in step (a), or (iv) the composition of step (b), whereby the added DNA from CHO cells reduces unspecific amplification in step (d). A second aspect of the invention is a composition comprising allantoic fluid from embryonated chicken eggs and DNA from CHO cells. A third aspect of the invention is a composition comprising (i) purified nucleic acids from a sample selected from the group consisting of amniotic fluid, a suspension of eukaryotic cells, and a supernatant from a suspension of eukaryotic cells, wherein the eukaryotic cells are derived from a human or an animal species other than Chinese hamster, and (ii) DNA from CHO cells which is free of prokaryotic DNA. A fourth aspect of the invention is the use of a composition according to the invention in a process for amplifying DNA of a prokyryotic contaminant from DNA isolated from a sample selected from the group consisting of amniotic fluid, a suspension of eukaryotic cells, and a supernatant from a suspension of eukaryotic cells. A fifth aspect of the invention is a kit comprising in separate containers (i) a lysis reagent, (ii) purified DNA from CHO cells at a predefined concentration, said DNA being free of prokaryotic DNA, and (iii) a first primer according to SEQ ID NO:1 and a second primer according to SEQ ID NO:2. Further disclosed is an improved method for performing a polymerase chain reaction (PCR), wherein a specific amplification product with a size in the range of about 100 bp to about 1500 bp is formed by DNA polymerase-catalyzed extension of a pair of oligonucleotide primers, said primers each hybridizing to a target sequence comprised in the genomic complement of the 16S-rRNA of one or more prokaryotic organism(s), wherein said pair of oligonucleotide primers is capable of forming in a PCR a specific amplification product from a first template, said PCR being conducted under predetermined conditions (R) in a reaction mixture (Q) with a predetermined composition, and said first template comprising genomic DNA of a first eukaryotic organism and genomic DNA of said one or more prokaryotic organism(s), and wherein in said PCR said pair of oligonucleotide primers does not form an amplification product from a second template under the same conditions (R) in the reaction mixture (Q), wherein in said second template genomic DNA of said one or more prokaryotic organism(s) is absent but genomic DNA of the first eukaryotic organism is comprised, the improved method comprising the steps of (a) providing said pair of oligonucleotide primers; (b) providing genomic DNA of said first eukaryotic organism; (c) providing a third template comprising genomic DNA of a second eukaryotic organism which is suspected to contain genomic DNA of said one or more prokaryotic organism(s); (d) mixing in said reaction mixture (Q) said primer pair of (a), said third template of (c), and a measured amount of said genomic DNA of said first eukaryotic organism of (b), and performing PCR under the conditions (R); wherein the formation of a non-specific PCR amplification product is suppressed.

### Detailed Description of the Invention

Certain terms are used with particular meaning, or are defined for the first time, in this description of the present invention. For the purposes of the present invention, the terms used are defined by their art-accepted definitions, when such exist, except that when those definitions conflict or partially conflict with the definitions set forth below. In the event of a conflict in definition, the meaning of a terms is first defined by any of the definitions set forth below.

The term "comprising" is used in the description of the invention and in the claims to mean "including, but not necessarily limited to".

The articles "a" and "an" are used herein to refer to one or to more than one (i.e. to at least one) of the grammatical object of the article. By way of example, "a microorganism" means one microorganism or more than one microorganism.

When designating a range of numerical values such as a concentration range, the range can be indicated by the word "between", followed by a first value n₁, the word "ahd", and a second value n₂. In addition, the designated range can be indicated by the expression "in the range of n₁ to n₂". If not stated otherwise, when a designated range is indicated, the lower boundary of the designated range is understood as being the value either equal to, or higher than the first value. The higher boundary of the designated range is understood as being either the value equal to, or lower than the second value". Thus, a value x in the designated range is given by n₁ ≤ x ≤ n₂.

Further, it is understood that the term "about" in combination with a numerical value n indicates a value x in the interval given by the numerical value ±5% of the value, i.e. n - 0.05 * n ≤ x ≤ n + 0.05 * n. In case the term "about" in combination with a numerical value n describes a preferred embodiment of the invention, the value of n is most preferred, if not indicated otherwise.

The term "sample" as used herein refers to a complex sample, more preferred a biological sample, i.e. a sample with biological material. The sample may contain a plurality of organic and inorganic compounds which are desired to be separated from nucleic acids comprised in the biological material. The term "sample" also encompasses an aqueous solution containing nucleic acids derived from other origins, e.g. from chemical or enzymatic reaction mixtures, or from a previous purification of biological sample material. The term biological sample, from which nucleic acids are purified, encompasses samples comprising viruses or bacterial cells, as well as isolated cells from multicellular organisms such as human and animal cells, as well as primary cultures of tissue and cultures of cell lines, as well as supernatants and rinses thereof. The present invention also encompasses biological samples such as a fluid from the human or animal body. Particularly, the sample can be whole blood, blood serum, blood plasma, cerebral fluid, sputum, stool, biopsy specimens, bone marrow, oral rinses, tissues, urine or mixtures thereof.

According to the invention, the preferred sample is a "liquid sample", i.e. the sample is in a fluidic state and the sample fluid comprises water and a biological material. The biological material preferably comprises cells or cellular components. Very much preferred, the liquid sample according to the invention is selected from the group consisting of a cell culture supernatant, a suspension of cultured cells and an amniotic fluid. Even more preferred, the amniotic fluid is from embryonated avian eggs.

For the purpose of the present invention, the meaning of the term "processing" or "processed" in combination with "liquid sample" is that the sample is treated by adding one or more compounds, and mixing the one or more compounds with the liquid sample, thereby resulting in a "processed sample". A preferred compound which can be used for such treatment is selected from the group consisting of a detergent, a surfactant, an organic solvent, a chaotropic agent, a protease, and a nuclease inhibiting agent. A "chaotropic agent" according to the present invention is any chemical substance which disturbs the ordered structure of liquid water. A chaotropic agent also facilitates unfolding, extension and dissociation of proteins (Dandliker, W., B., and de Saussure, V., A., In: The Chemistry of Biosurfaces, Hair, M., L., ed., Marcel Dekker, Inc. New York (1971) p. 18).

A preferred processed sample according to the invention is a lysate. A "lysate" or a "lysed sample" can be obtained from a comprising cells, preferably microbial cells, and very much preferred bacterial cells, wherein the structural integrity of a substantial portion of the cells present is disrupted. To this end, the cell wall, if present, has to be destroyed. To release the contents of disrupted bacterial cells the material is treated with certain agents to disintegrate, make porous, dissolve, degrade or denature the cell walls of the microbial cells. In addition, the cellular membranes have to be destroyed. To release the contents of cells, tissue or, more generally, from the particles which are comprised in the biological sample, the material may be treated with enzymes or with chemicals to dissolve, degrade or denature the cellular walls and cellular membranes of such organisms. In any such case the lysis ("lytic") process will also disrupt the structural integrity of the bacterial contaminant, thereby liberating nucleic acids of the contaminant.

For the lytic process it is common to use chaotropic agents such as a guanidinium salt and/ or anionic, cationic, zwitterionic or non-ionic detergent when nucleic acids are set free in the process. It is also an advantage to use proteases which rapidly degrade enzymes with nucleolytic activity and other unwanted proteins. In case there remains particulate, i.e. undissolved matter of the sample material following the lysis process, the particulate matter can be separated from the lysate to result in a cleared lysate. This can be done, e.g., by way of filtering or centrifugation. Thus, the term "lysate" encompasses a cleared lysate.

"Purification of nucleic acids" from a processed sample can be done using a wide variety of methods which are standard techniques. These may include precipitation of nucleic acids from an aqueous solution, e.g. using an alcohol (precipitation with ethanol or isopropanol being well-known to the art), and isolation of the precipitate. Other methods include adsorption of nucleic acids onto a solid phase (e.g. with an oxidic surface such as silica), separating the solid phase, followed by eluting the nucleic acids from the solid phase.

The "polymerase chain reaction" ("PCR") is a technique for amplifying a single or few copies of a particular target DNA sequence across several orders of magnitude, wherein copies of the DNA sequence are generated. PCR relies on thermal cycling, consisting of cycles of repeated heating and cooling of the reaction mixture, thereby effecting DNA melting and enzymatic replication of the DNA. Primers (DNA oligonucleotides) containing sequences complementary to the target DNA sequence along with a DNA polymerase are key components to enable selective and repeated amplification. As PCR progresses, the DNA generated is itself used as a template for replication, setting in motion a chain reaction in which the DNA template is exponentially amplified. A "reaction mixture" in this regard comprises a DNA polymerase, dNTP, ions, buffer and all other compounds necessary to effect extension of the primers hybridized (annealed) to the target DNA sequence.

The inventor has concluded that in the method disclosed by Eldering, J.A., et al. *(supra)* the PCR conditions which are key to determining the presence or absence of a bacterial contaminant are optimized exclusively for CHO (Chinese hamster ovary) cell cultures and/or culture supernatants. In addition, the inventor has observed, that the primer pair according to SEQ ID NO:1 and SEQ ID NO:2 occasionally produces irregular amplificates or artifacts (i.e. non-specific amplification of fragments differing in size from the desired target DNA fragment). This is especially the case when DNA prepared from sample material from cultures of cells derived from other animal species or humans is used as template. In such a setting the fundamental observation on which the present invention is based is that formation of the PCR artifacts can be suppressed when the PCR is performed in the presence of CHO cell DNA.This desired technical effect, however, requires the CHO cell DNA to be free of contamination with bacterial DNA which can be amplified by the said primer pair. Therefore, in the broadest sense, a first disclosure herein is
an improved method for performing a polymerase chain reaction (PCR),
wherein a specific amplification product with a size in the range of about 100 bp to about 1500 bp is formed by DNA polymerase-catalyzed extension (PCR) of a pair of oligonucleotide primers, said primers each hybridizing to a target sequence comprised in the genomic complement of the 16S-rRNA of one or more prokaryotic organism(s),
wherein said pair of oligonucleotide primers is capable of forming in a PCR a specific amplification product from a first template, said PCR being conducted under predetermined conditions (R) in a reaction mixture with a predetermined composition (Q), and said first template comprising genomic DNA of a first eukaryotic organism and genomic DNA of said one or more prokaryotic organism(s),
and wherein in said PCR said pair of oligonucleotide primers does not form an amplification product from a second template under the same conditions (R) in the reaction mixture (Q), wherein in said second template genomic DNA of said one or more prokaryotic organism(s) is absent but genomic DNA of the first eukaryotic organism is comprised,

### the improved method comprising the steps of

(a) providing said pair of oligonucleotide primers;
(b) providing genomic DNA of said first eukaryotic organism;
(c) providing a third template comprising genomic DNA of a second eukaryotic organism which is suspected to contain genomic DNA of said one or more prokaryotic organism(s);
(d) mixing in the reaction mixture (Q) said primer pair of (a), said third template of (c), and a measured amount of said genomic DNA of said first eukaryotic organism of (b), and performing PCR under the conditions (R);
wherein the formation of a non-specific PCR amplification product is suppressed.

In the context of the present invention, the conditions (R) of the PCR reflect parameters including temperature regime, incubation times, number of PCR cycles and other physical parameters known to the skilled person in the field of PCR. The reaction mixture (Q) comprises all components of the final mixture with which the PCR process is conducted, including all compounds needed for primer elongation. The reaction mixture (Q) also includes the pair of primers, each at its respective predetermined concentration. For the sake of clarity, the reaction mixture (Q) in this sense does not comprise the template DNA which is added separately. The template DNA is added such that in the final mixture (i.e. after addition of the template DNA) the concentrations of all other components are identical, no matter which template is used.

According to the invention a template containing genomic DNA of another organism but CHO cells additionally has to contain CHO cell DNA in a measured amount, i.e. at a predetermined concentration.

In a preferred embodiment of the invention, the pair of oligonucleotides hybridizes to a plurality of prokaryotic species, and between 0 to 3 mismatches may occur in the hybridization of the primers with their target region of the 16S-rRNA gene. In any such case, any mismatch (if present) excludes the terminal nucleotide providing the 3'-OH group of the respective oligonucleotide.

In a preferred embodiment of the invention, said one or more prokaryotic organism(s) is a species selected from the group consisting of a Mollicutes species, a Bacillus species, a Clostridium species, a Corynebacterium species, a Micrococcus species, a Staphylococcus species, and a Streptococcus species. Even more preferred, the species is a Mollicutes species. Even more preferred, the species is a Mycoplasma species, even more preferred a species selected from the group consisting of M. hyorhinis, M. arginini, M. pneumoniae, M. fermentans, M. orale, and M. pirium, or the species is an Acholeplasma species, even more preferred Acholeplasma laidlawii, or the species is a Spiroplasma species, even more preferred Spiroplasma mirium.

In a very much preferred embodiment of the invention, said first eukaryotic organism is a CHO cell or a culture comprising a prurality of CHO cells. Even more preferred, said pair of primers comprises either one of SEQ ID NO:1 and SEQ ID NO:2, or both.

In an even more preferred embodiment of the invention, the the conditions (R) and the reaction mixture (Q) are those of the MYCOTOOL assay by Roche Diagnostics GmbH, Mannheim (Germany) described in the manuals of the MYCOTOOL test kits.

Further preferred embodiments of the present disclosure are given in the following list of items:
1. An improved method for determining the presence or absence of a bacterial contaminant in a sample with biological material, the bacterial contaminant being selected from the group consisting of Mycoplasma hyorhinis, Mycoplasma arginini, Mycoplasma pneumoniae, Mycoplasma fermentans, Mycoplasma orale. Mycoplasma pirium, Acholeplasma laidlawii and Spiroplasma mirium, said method comprising the steps of
   (a) processing the sample and purifying nucleic acids from the processed sample, followed by
   (b) forming a composition for a PCR-based amplification reaction , the composition including
      - a first primer according to SEQ ID NO: 1.
      - a second primer according to SEQ ID NO:2, and
      - the purified nucleic acids of step (a) or a measured fraction thereof as a template; followed by
   (c) performing a polymerase chain reaction (PCR) with the composition of step (b), whereby a target sequence comprised in a prokaryotic 16S-rRNA gene, if present in the template, is amplified; followed by
   (d) detecting the presence or absence of an amplified target sequence, whereby the presence of said amplified target sequence indicates the presence of the bacterial contaminant in the sample, and the absence of said amplified target sequence indicates the absence of the bacterial contaminant in the sample;
   **the improvement being characterized in that** relative to the volume of the sample a predetermined amount of DNA from CHO cells is added to (i) the sample, or (ii) the processed sample of step (a), or (iii) the purified nucleic acids obtained in step (a), or (iv) the composition of step (b) whereby the added DNA from CHO cells reduces unspecific amplification in step (d).
2. The method of item 1, **characterized in that** the predetermined amount of DNA per ml of the liquid sample is the DNA content from about 5 x 10⁶ CHO cells.
3. The method according to any of the items 1 and 2, **characterized in that** the liquid sample is selected from the group consisting of cell culture medium with cultured cells, cell-free culture supernatant, and amniotic fluid.
4. The method according to item 3, **characterized in that** the liquid sample is amniotic fluid.
5. The method according to item 4, characterized in that the amniotic fluid is from embryonated eggs.
6. A composition comprising allantoic fluid from embryonated chicken eggs and DNA from CHO cells.
7. The composition according to any item 6, further comprising a lysis reagent selected from a chaotropic agent and a protease.
8. A composition comprising (i) purified nucleic acids from a sample selected from the group consisting of amniotic fluid, a suspension of eukaryotic cells, and a supernatant from a suspension of eukaryotic cells, wherein the eukaryotic cells are derived from a human or an animal species other than Chinese hamster, and (ii) DNA from CHO cells which is free of prokaryotic DNA.
9. The composition according to item 8, further comprising a first primer according to SEQ ID NO:1, a second primer according to SEQ ID NO:2, nucleotide triphosphates, and a thermostable DNA polymerase.
10. The composition according to item 10, further comprising an intercalating dye.
12. A kit comprising in separate containers (i) a lysis reagent, (ii) purified DNA from CHO cells at a predefined concentration, said DNA being free of prokaryotic DNA, and (iii) a first primer according to SEQ ID NO:1 and a second primer according to SEQ ID NO:2.
13. The use of a composition according to any of the items 10 to 12 for amplifying DNA of a prokyryotic contaminant from DNA isolated from a sample selected from the group consisting of amniotic fluid, a suspension of eukaryotic cells, and a supernatant from a suspension of eukaryotic cells.
14. An improved method for performing an improved polymerase chain reaction (PCR),
   wherein a specific amplification product with a size in the range of about 100 bp to about 1500 bp is formed by DNA polymerase-catalyzed extension of a pair of oligonucleotide primers, said primers each hybridizing to a target sequence comprised in the genomic complement of the 16S-rRNA of one or more prokaryotic organism(s),
   wherein said pair of oligonucleotide primers is capable of forming in a PCR (P) a specific amplification product from a first template, said PCR (P) being conducted under predetermined conditions (R) in a reaction mixture (Q) with a predetermined composition, and said first template comprising genomic DNA of a first eukaryotic organism and genomic DNA of said one or more prokaryotic organism(s),
   and wherein in P said pair of oligonucleotide primers does not form an amplification product from a second template under the same conditions (R) in the reaction mixture (Q), wherein in said second template genomic DNA of said one or more prokaryotic organism(s) is absent but genomic DNA of the first eukaryotic organism is comprised,
   the improved method comprising the steps of
   (a) providing said pair of oligonucleotide primers and genomic DNA of said first eukaryotic organism;
   (b) providing a third template comprising genomic DNA of a second eukaryotic organism which is suspected to contain genomic DNA of said one or more prokaryotic organism(s);
   (c) mixing said primer pair of (a), said third template of (b), and a measured amount of said genomic DNA of said first eukaryotic organism in the reaction mixture (Q) and performing PCR under the conditions (R);
   wherein the formation of a non-specific PCR amplification product is suppressed.

### Description of the Sequence Listing

- **SEQ ID NO:1**: universal primer (forward) for the detection of Mycoplasma and related species; previously disclosed in Wong-Lee, J.G., and Lovett, M., "Rapid and sensitive PCR method for identification of Mycoplasma species in tissue culture" in: Diagnostic molecular microbiology - principles and applications; Persing, DH, Smith, TF, Tenover, FC, White, TJ (editors) Washington, DC, American Society for Microbiology (1993) 257-260, and Eldering, J.A., et al., Biologicals 32 (2004) 183-193.
- **SEQ ID NO:2**: universal primer (reverse) for the detection of Mycoplasma and related species; previously disclosed in Wong-Lee, J.G., et al. (*supra*) and Eldering, J.A., et al. (*supra*).
- **SEQ ID NO:3**: forward primer specific for a target sequence in the Glyceraldehyde 3-phosphate dehydrogenase gene (control sequence).
- **SEQ ID NO:4**: reverse primer specific for a target sequence in the Glyceraldehyde 3-phosphate dehydrogenase gene (control sequence).

### Description of the Figures

| **Figure 1** | **Lane** | **Comment / dilution** |
|---|---|---|
| | 1-5 | GAPDH control PCR; DNA isolated from MDCK cells, no CHO cell DNA, spiked with A. laidlawii DNA |
| | 1 | undiluted |
| | 2 | 10⁻¹ |
| | 3 | 10⁻² |
| | 4 | 10⁻³ |
| | 5 | 10⁻⁴ |
| | 6 | Size marker (50 bp steps) |
| | 7-11 | GAPDH control PCR; DNA isolated from MDCK cells, no CHO cell DNA, not spiked |
| | 7 | 10⁻¹ |
| | 8 | 10⁻² |
| | 9 | 10⁻³ |
| | 10 | 10⁻⁴ |
| | 11 | Size marker (50 bp steps) |

| **Figure 2** | **Lane** | **Comment / dilution** |
|---|---|---|
| | 1-5 | GAPDH control PCR; DNA isolated from MDCK cells, no CHO cell DNA, spiked with A. laidlawii DNA |
| | 1 | undiluted |
| | 2 | 10⁻¹ |
| | 3 | 10⁻² |
| | 4 | 10⁻³ |
| | 5 | 10⁻⁴ |
| | 6 | Size marker (50 bp steps) |
| | 7-11 | GAPDH control PCR; DNA isolated from MDCK cells, no CHO cell DNA, not spiked |
| | 7 | 10⁻¹ |
| | 8 | 10⁻² |
| | 9 | 10⁻³ |
| | 10 | 10⁻⁴ |
| | 11 | Size marker (50 bp steps) |

| **Figure 3** | **Lane** | **Comment / dilution** |
|---|---|---|
| | 1-8 | Primers as in SEQ ID NO:1 and SEQ ID NO:2; DNA isolated from ASC, spiked with M. orale DNA, no CHO cell DNA |
| | 1-8 | Primers as in SEQ ID NO:1 and SEQ ID NO:2; PCR with 8 independently drawn aliquots |
| | 6 | The size of the band is in line with the size range observed for a specific target DNA amplification product |

| **Figure 4** | **Lane** | **Comment / dilution** |
|---|---|---|
| | 1-8 | Primers as in SEQ ID NO:1 and SEQ ID NO:2; DNA isolated from ASC, spiked with M. orale DNA, with CHO cell DNA added |
| | 1-8 | Primers as in SEQ ID NO:1 and SEQ ID NO:2; PCR with 8 independently drawn aliquots |
| | 9-12 | Primers as in SEQ ID NO:1 and SEQ ID NO:2; PCR. with buffer ("no template" control) |
| | 13 | Size marker (50 bp steps) |

| **Figure 5** | **Lane** | **Comment / dilution** |
|---|---|---|
| | 1-8 | Primers as in SEQ ID NO:1 and SEQ ID NO:2; Tris buffer spiked with A. laidlawii DNA, no CHO cell DNA |
| | 1-4 | 10 cfu A. laidlawii DNA |
| | 5-8 | 1 cfu A. laidlawii DNA |
| | 9-12 | Primers as in SEQ ID NO:1 and SEQ ID NO:2; Tris buffer, not spiked, no CHO cell DNA |
| | 13, 14 | Primers as in SEQ ID NO:1 and SEQ ID NO:2; Tris buffer, about 10 copies of positive control plasmid (part of MYCOTOOL kit) per PCR reaction mixture, no CHO cell DNA |
| | 15 | Size marker (50 bp steps) |

| **Figure 6** | **Lane** | **Comment / dilution** |
|---|---|---|
| | 1-8 | Primers as in SEQ ID NO:1 and SEQ ID NO:2; Tris buffer spiked with A. laidlawii DNA, with CHO cell DNA added |
| | 1-4 | 10 cfu A. laidlawii DNA |
| | 5-8 | 1 cfu A. laidlawii DNA |
| | 9-12 | Primers as in SEQ ID NO:1 and SEQ ID NO:2; Tris buffer, not spiked, with CHO cell DNA added |
| | 13, 14 | Primers as in SEQ ID NO:1 and SEQ ID NO:2; Tris buffer, about 10 copies of positive control plasmid (part of MYCOTOOL kit) per PCR reaction mixture, with CHO cell DNA added |
| | 15 | Size marker (50 bp steps) |

| **Figure 7** | **Lane** | **Comment / dilution** |
|---|---|---|
| | 1-4 | Primers as in SEQ ID NO:1 and SEQ ID NO:2; Vero cell suspension spiked with A. laidlawii DNA, no CHO cell DNA |
| | 1-4 | 10 cfu A. laidlawii DNA |
| | 5-8 | Primers as in SEQ ID NO:1 and SEQ ID NO:2; Vero cell suspension, not spiked, no CHO cell DNA |
| | 9, 10 | Primers as in SEQ ID NO:1 and SEQ ID NO:2; Tris buffer, about 10 copies of positive control plasmid (part of MYCOTOOL kit) per PCR reaction mixture, no CHO cell DNA |
| | 11, 12 | Primers as in SEQ ID NO:1 and SEQ ID NO:2; PCR with buffer ("no template" control) |
| | 13 | Size marker (50 bp steps) |

| **Figure 8** | **Lane** | **Comment / dilution** |
|---|---|---|
| | 1-4 | Primers as in SEQ ID NO:1 and SEQ ID NO:2; Vero cell suspension spiked with A. laidlawii DNA, with CHO cell DNA added |
| | 1-4 | 3 cfu A. laidlawii DNA |
| | 5-8 | Primers as in SEQ ID NO:1 and SEQ ID NO:2; Vero cell suspension, not spiked, with CHO cell DNA added |
| | 9, 10 | Primers as in SEQ ID NO:1 and SEQ ID NO:2; Tris buffer, about 10 copies of positive control plasmid (part of MYCOTOOL kit) per PCR reaction mixture, with CHO cell DNA added |
| | 11, 12 | Primers as in SEQ ID NO:1 and SEQ ID NO:2; PCR with buffer ("no template" control) |
| | 13 | Size marker (50 bp steps) |

| **Figure 9** | **Lane** | **Comment / dilution** |
|---|---|---|
| | 1-4 | Primers as in SEQ ID NO:1 and SEQ ID NO:2; allantois fluid "inoculated" with A. laidlawii, no CHO cell DNA |
| | 1-4 | 3 cfu A. laidlawii DNA |
| | 5-8 | Primers as in SEQ ID NO:1 and SEQ ID NO:2; allantois fluid, not inoculated, no CHO cell DNA |
| | 9, 10 | Primers as in SEQ ID NO:1 and SEQ ID NO:2; Tris buffer, about 10 copies of positive control plasmid (part of MYCOTOOL kit) per PCR reaction mixture, no CHO cell DNA |
| | 11 | Size marker (50 bp steps) |
| | The arrow indicates the region of the gel in which non-specifically amplified PCR products migrate | |

| **Figure 10** | **Lane** | **Comment / dilution** |
|---|---|---|
| | 1-4 | Primers as in SEQ ID NO:1 and SEQ ID NO:2; allantois fluid "inoculated" with A. laidlawii, 2.5 µg/50 µl CHO cell DNA |
| | 1-4 | 3 cfu A. laidlawii DNA |
| | 5-8 | Primers as in SEQ ID NO:1 and SEQ ID NO:2; allantois fluid, not inoculated, 2.5 µg/50 µl CHO cell DNA |
| | 9, 10 | Primers as in SEQ ID NO:1 and SEQ ID NO:2; Tris buffer, about 10 copies of positive control plasmid (part of MYCOTOOL kit) per PCR reaction mixture, no CHO cell DNA |
| | 11 | Size marker (50 bp steps) |
| | The arrow indicates the region of the gel in which non-specifically amplified PCR products migrate | |

| **Figure 11** | **Lane** | **Comment / dilution** |
|---|---|---|
| | 1-4 | Primers as in SEQ ID NO:1 and SEQ ID NO:2; allantois fluid "inoculated" with A. laidlawii, 5 µg/50 µl CHO cell DNA |
| | 1-4 | 3 cfu A. laidlawii DNA |
| | 5-8 | Primers as in SEQ ID NO:1 and SEQ ID NO:2; allantois fluid, not inoculated, 5 µg/50 µl CHO cell DNA |
| | 9, 10 | Primers as in SEQ ID NO:1 and SEQ ID NO:2; PCR with buffer ("no template" control) |
| | 11 | Size marker (50 bp steps) |
| | The arrow indicates the region of the gel in which non-specifically amplified PCR products migrate | |

| **Figure 12** | **Lane** | **Comment / dilution** |
|---|---|---|
| | 1-4 | Primers as in SEQ ID NO:1 and SEQ ID NO:2; allantois fluid "inoculated" with A. laidlawii, 10 µg/50 µl CHO cell DNA |
| | 1-4 | 3 cfu A. laidlawii DNA |
| | 5-8 | Primers as in SEQ ID NO:1 and SEQ ID NO:2; allantois fluid, not inoculated, 10 µg/50 µl CHO cell DNA |
| | 9, 10 | Primers as in SEQ ID NO:1 and SEQ ID NO:2; PCR with buffer ("no template" control) |
| | 11 | Size marker (50 bp steps) |
| | The arrow indicates the region of the gel in which non-specifically amplified PCR products migrate | |

| **Figure 13** | **Lane** | **Comment / dilution** |
|---|---|---|
| | 1-8 | Primers as in SEQ ID NO:1 and SEQ ID NO:2; Tris buffer spiked with A. laidlawii DNA, |
| | 1-4 | 3 cfu A. laidlawii DNA, no calf thymus DNA |
| | 5-8 | 1 cfu A. laidlawii DNA, with calf thymus DNA added |
| | 9-12 | Primers as in SEQ ID NO:1 and SEQ ID NO:2; Tris buffer unspiked, with calf thymus DNA added |
| | 13, 14 | Primers as in SEQ ID NO:1 and SEQ ID NO:2; Tris buffer, about 10 copies of positive control plasmid (part of MYCOTOOL kit) per PCR reaction mixture, no calf thymus DNA |
| | 15 | Size marker (50 bp steps) |

| **Figure 14** | **Lane** | **Comment / dilution** |
|---|---|---|
| | 1-8 | Primers as in SEQ ID NO:1 and SEQ ID NO:2; allantois fluid "inoculated" with A. laidlawii, with calf thymus DNA added |
| | 1-4 | 3 cfu A. laidlawii DNA |
| | 5-8 | Primers as in SEQ ID NO:1 and SEQ ID NO:2; allantois fluid, not inoculated, with calf thymus DNA added |
| | 9-12 | Primers as in SEQ ID NO:1 and SEQ ID NO:2; Tris buffer, no inoculum, with calf thymus DNA added |
| | 13, 14 | Primers as in SEQ ID NO:1 and SEQ ID NO:2; PCR with buffer ("no template" control) |
| | 15 | Size marker (50 bp steps) |

### Example 1

### The MYCOTOOL kit and assay

The MYCOTOOL PCR Mycoplasma Detection Kit is an in vitro nucleic acid amplification test optimized for the detection of bacteria belonging to the Mollicutes. These include Mycoplasma hyorhinis, M. arginini, M. pneumoniae, M. fermentans, M. orale, M. pirium, M. salivarum, M. hominis, M. synoviae, Spiroplasma mirium, S. citri, and Acholeplasma laidlawii.

The MycoTool Kit comprises two subkits: Subkit 1 ("Detection Prep Kit"; Roche Applied Science Catalog No. 05184592001) and Subkit 2 ("Detection Amplification Kit"; Roche Applied Science Catalog No. 05184240001).

The kit was used exactly according to the instructions of the manufacturer.

If not stated otherwise, a liquid sample selected from (i) cell culture supernatant, (ii) a suspension of cultured cells and (iii) amniotic fluid was lysed by adding an aqueous buffer containing a guanidinium salt and proteinase K, mixing the buffer with the sample, and incubating the mixture to effect lysis.

After lysis, typically 10-250 µg per 1 ml sample CHO cell DNA was added to the lysate and mixed. The CHO cell DNA was free of any contaminating prokaryotic DNA as tested separately. Subsequently the nucleic acids were precipitated from the mixture by adding alcohol. The precipitate was recovered by centrifugation, washed with 70% ethanol and dried. The dried pellet was dissolved in the prescribed buffer and subjected to PCR analysis.

As an internal control the MYCOTOOL kit also includes a primer pair for the GAPDH housekeeping gene.

Prior to PCR amplification, the risk of amplicon contamination was reduced by applying uracil-N-glycosylase enzyme (component of the kit).

In each Example below PCR was performed exactly according to the instructions by the manufacturer. PCR products were electrophoresed on polyacrylamide gels under standard conditions. Bands were visualized with the RESOLIGHT compound and UV illumination, band detection was at 520 nm.

The primers of the MYCOTOOL kit used for detection of Mollicutes are those of SEQ ID NO:1 and SEQ ID NO:2. Control primers specific for GAPDH are also provided (SEQ ID NO:3 and SEQ ID NO:4).

Each MYCOTOOL kit further comprises a control plasmid containing Mycoplasma DNA, however producing a PCR fragment with a discernably different size compared to the PCR fragments amplified from isolated bacterial DNA (positive control). To this end, reference is made to Eldering, J.A., et al., Biologicals 32 (2004) 183-193, who disclose the plasmid.

### Example 2

### DNA from Mollicutes species for spiking of samples

All samples used in the present Examples were from cultures free of prokaryotic contaminants.

Instead of sample material infected with a Mollicutes species, DNA prepared from Acholeplasma laidlawii or Mycoplasma orale was spiked to either sample material prior to lysis, or to isolated DNA prepared from sample material (if not indicated otherwise). For the purpose of spiking DNA was prepared from reference cultures (standard conditions) of Acholeplasma laidlawii (ATCC 27556) and Mycoplasma orale (ATCC 23714). For each culture the cell titer expressed as colony forming units (cfu) was determined. Quantities of spiked DNA which were applied reflected the number of cfu determined for the respective culture from which the DNA was prepared.

Mollicutes DNA as indicated above was used in the spiking experiments described below. The size of a typical specific PCR fragment amplified from Mollicutes DNA (specific amplification product) was in the range of about 430-470 bp.

### Example 3

### Detection of GAPDH genomic target sequences in samples from MDCK cell culture (suspended cells)

A culture of MDCK cells free of any prokaryotic organisms and having a cell titer of 0,79·10⁶ cells/ml was used. Acholeplasma laidlawii DNA was added to the sample at a concentration of 3 colony forming units (cfu) per 1 ml sample. Nucleic acids were isolated from the spiked sample material as specified in the instruction manual of the MYCOTOOL kit (see also Example 1). Two different nucleic acid preparations were made, the first without addition of CHO cell DNA, the second with the addition of CHO cell DNA (50 mg per 1 ml sample) to the sample material.

The preparation was repeated, however without spiking Acholeplasma laidlawii DNA to the sample. Again, nucleic acids were prepared with or without CHO cell DNA.

Several dilutions of the DNA preparations were made in TE buffer. An aliquot of each dilution was subjected to GAPDH-specific PCR according to the MYCOTOOL instruction manual.

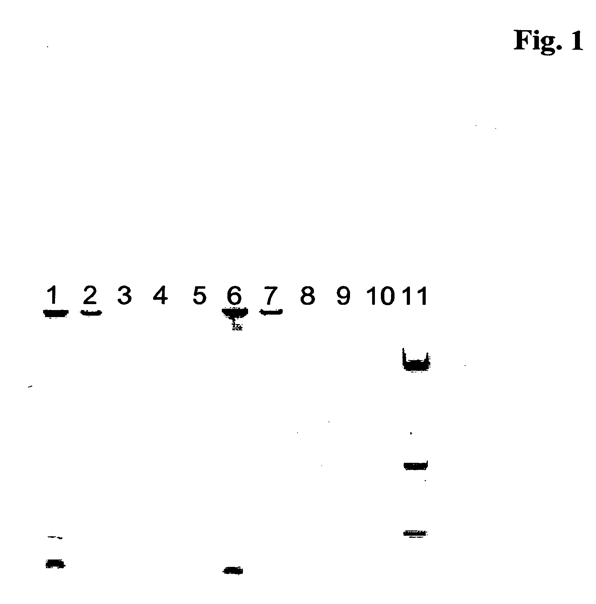

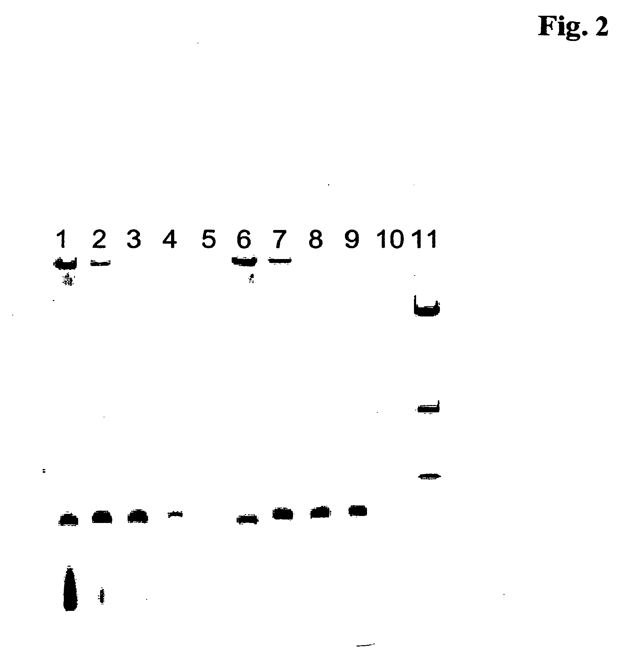

Figures 1 and 2 show the gels with the bands indicating the PCR products which were obtained. Figure 1 depicts the results obtained without CHO cell DNA added, Figure 2 sows the PCR products obtained with CHO cell DNA added.

It could clearly be observed that with CHO DNA added the GAPDH control bands were detectable even when PCR was performed with 10⁻⁴ dilutions.

### Example 4

### Detection of target sequences in the 16S-rRNA complement of prokaryotic DNA in spiked samples from culture supernatant of adipose stem cells (ASC)

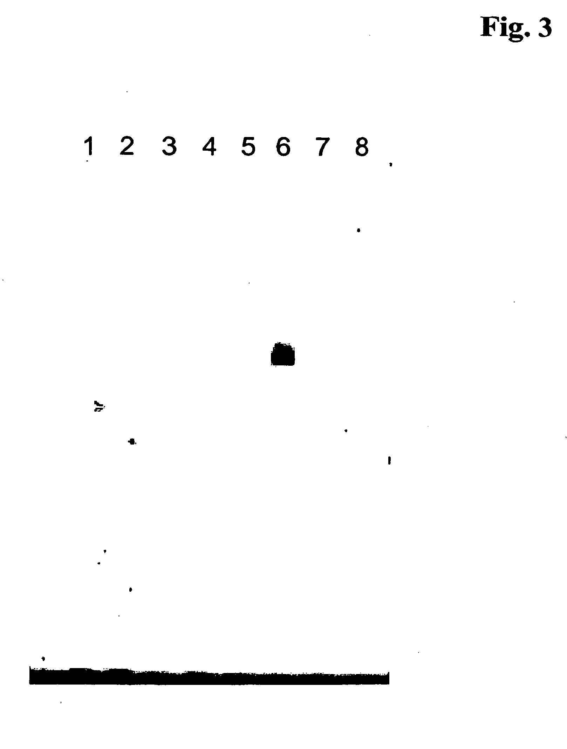

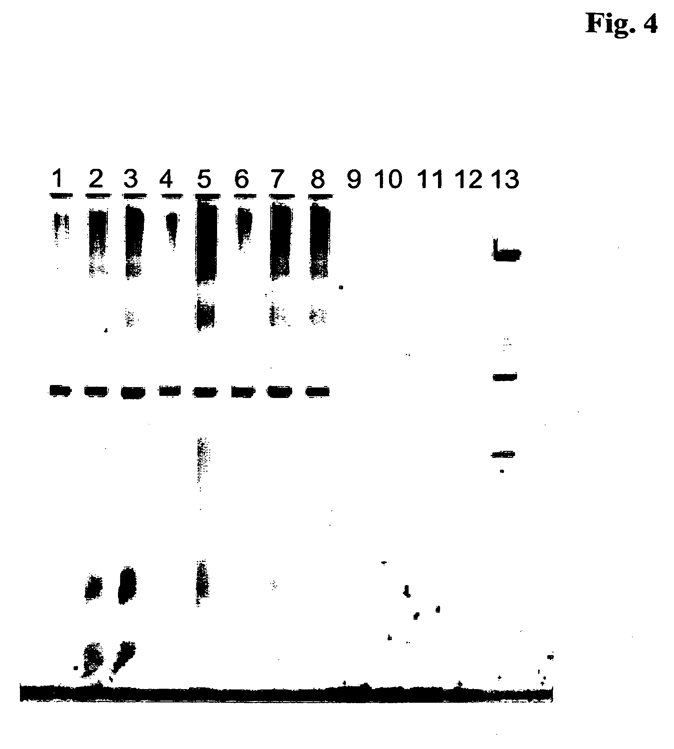

ASC free of any prokaryotic organisms were sedimented by centrifugation. The cleared supernatant (cell culture medium) was used for DNA isolation as specified in the instruction manual of the MYCOTOOL kit (see also Example 1). Prior to the lysis step, Mycoplasma orale DNA was added to the sample at a concentration of 3 colony forming units (cfu) per 1 ml sample. The sample was then divided into two equal volumes. To one aliquot CHO cell DNA was added at a concentration of 100 mg per 1 ml of supernatant. Total DNA was isolated from both aliquots separately. Several PCR reactions were performed with aliquots of each DNA preparation. Figures 3 and 4 show the gels with the amplification products obtained by PCR and after electrophoresing the DNA fragments.

Clearly, the addition of CHO cell DNA made the MYCOTOOL test more robust in that Mycoplasma DNA was detected in all spiked samples tested. On the other hand, without CHO cell DNA only 1 of 8 PCR reactions successfully amplified a fragment specific for the Mycoplasma target DNA.

In the case where CHO cell DNA is absent it can be seen (Figure 3) that non-specific PCR fragments are substantially absent (lane 1 in Figure 3 being a possible exception). This underlines the inventor's observation that PCR artifacts are not commonly produced by the MYCOTOOL assay. Interestingly, Addition of CHO cell DNA also does not lead to unspecific amplification products as can be seen in Figure 4.

### Example 5

### Detection of target sequences in the 16S-rRNA complement of prokaryotic DNA in spiked aqueous buffer

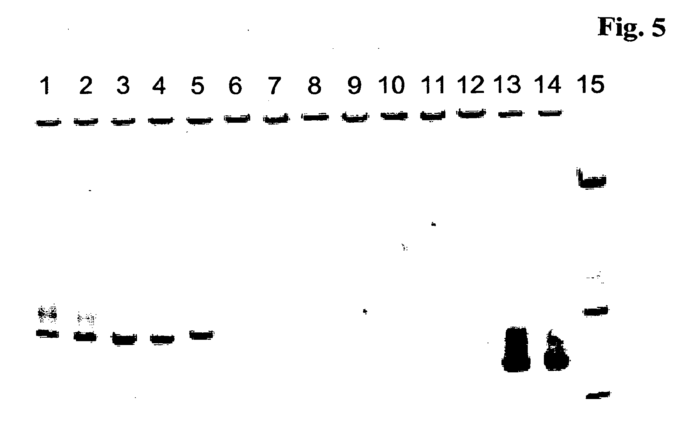

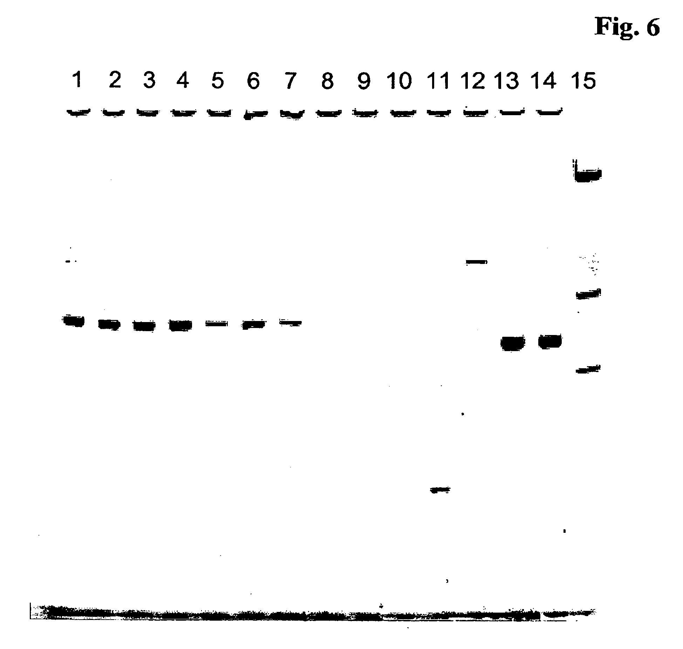

10 mM TrisHCl buffer pH 7.5 free of any contaminants was spiked with A. laidlawii DNA at a concentration of 1 or 10 cfu per 1 ml of buffer. Spiked and unspiked buffer was mixed with CHO cell DNA to yield a concentration of 80 µg per 1 ml of buffer. In addition spiked and unspiked buffer without CHO cell DNA was prepared. From each spiked and unspiked preparation DNA was prepared according to the MYCOTOOL protocol. Several PCR reactions were performed with aliquots of each DNA preparation. In addition, PCR was performed with an aliquot of Tris buffer containing about 10 copies of the control plasmid which is part of the MYCOTOOL kit (positive control). Figures 5 and 6 show the gels with the amplification products obtained by PCR and after electrophoresing the DNA fragments.

While the PCR had no problems detecting the A. laidlawii DNA corresponding to 10 cfu, detection of 1 cfu was markedly improved in the presence of CHO cell DNA.

The lanes with the positive control plasmid illustrate the discernible size difference compared with the specific amplification products of A. laidlawii target sequence.

### Example 6

### Detection of target sequences in the 16S-rRNA complement of prokaryotic DNA in spiked samples from culture Vero cell culture (suspended cells)

Suspensions of Vero cells with a cell titer in the range of 10⁵ to 10⁶ cells per ml from a culture which was free of any prokaryotic organisms were spiked with A. laidlawii or M. orale DNA at a concentration of 3 or 10 cfu per 1 ml of cell suspension. To the culture spiked with 3 cfu/ml CHO cell DNA was added at a concentration of 10, 30, 50, 70, 85, 100, and 150 µg per 1 ml of cell suspension.

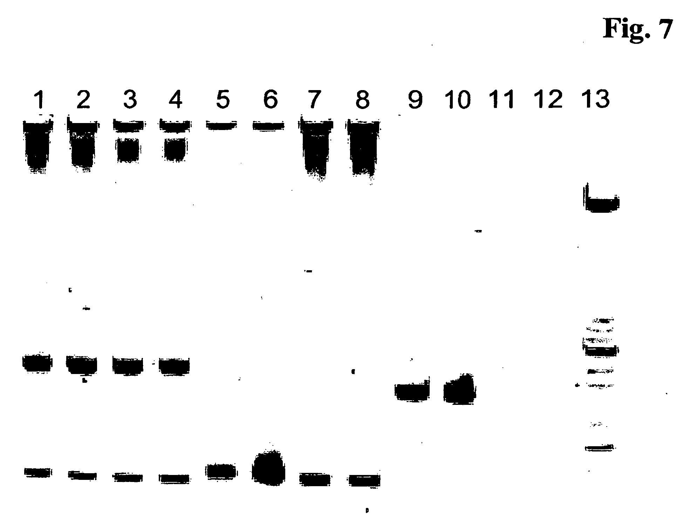

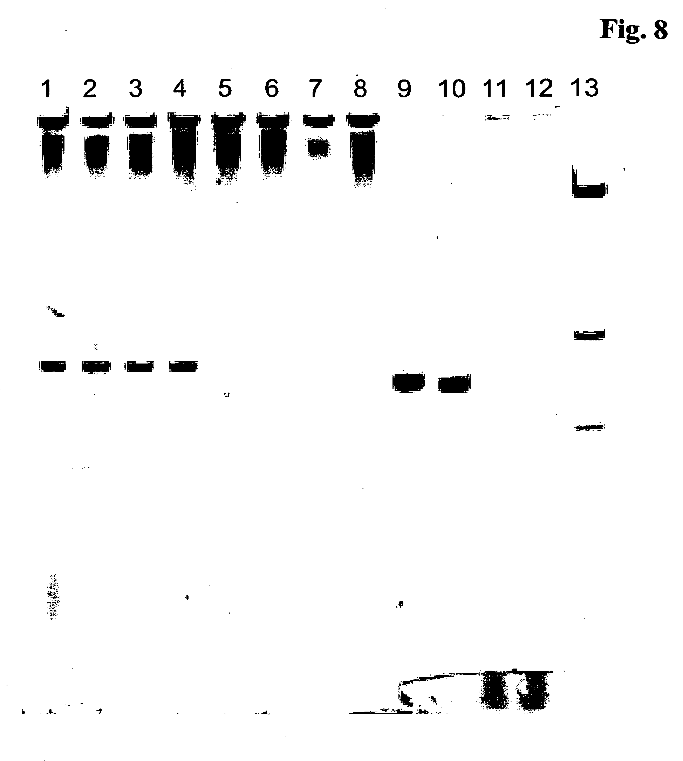

From each spiked suspension as well as from unspiked suspensions of Vero cells alone DNA was prepared according to the MYCOTOOL protocol. Several PCR reactions were performed with aliquots of each DNA preparation. In addition, PCR was performed with an aliquot of Tris buffer (10 mM TrisHCl buffer pH 7.5) containing about 10 copies of the control plasmid which is part of the MYCOTOOL kit (positive control). Figures 7 and 8 show the gels with the amplification products obtained by PCR and after electrophoresing the DNA fragments. The samples shown in Figure 8 contained CHO cell DNA at a concentration of 150 µg per 1 ml of cell suspension. Comparable results were obtained when using the concentrations of 10, 30, 50, 70, 85, and 100 µg CHO cell DNA per 1 ml of cell suspension.

Notably, some non-specific (artifact) PCR products were obtained in the absence of CHO cell DNA (see Figure 7). The similarity in some cases of the size of these artifact bands to the size of the specific amplification products (lanes 1-4 in Figure 7) was noted. Surprisingly, no unspecific PCR amplification products were produced in in the presence of CHO cell DNA (Figure 8, lanes 1-4).

### Example 7

### Detection of target sequences in the 16S-rRNA complement of prokaryotic DNA in "inoculated" samples of allantois fluid from embryonated chicken eggs

Allantois fluid free of mycoplasmal contamination was harvested from embryonated chicken eggs (9 to 11 days) which were inoculated with a viral vaccine strain.

A liquid culture of A. laidlawii with a titer of 2.45 x 10³ cfu was diluted with TE buffer 1:1,000. An aliquot of this dilution was diluted 1:10 with allantois fluid. A volume of 12.2 µl of this 1:10,000 inoculum was added per 1 ml of allantois fluid to result in an equivalent titer of 3 cfu per 1 ml of inoculated allantois fluid. The inoculated allantois fluid was subjected to DNA isolation without further incubation, i.e. the bacteria were not allowed to grow in the allantois fluid.

Four different samples were provided: (i) inoculated allantois fluid without CHO cell DNA, (ii) non inoculated allantois fluid without CHO cell DNA, (iii) inoculated allantois fluid with CHO cell DNA added, and (iv) non inoculated allantois fluid with CHO cell DNA added. In the samples of (iii) and (iv) the concentration of the CHO cell DNA was 208 mg per 1 ml of allantois fluid.

From each inoculated and non-inoculated sample (i-iv) DNA was prepared according to the MYCOTOOL protocol. Several PCR reactions were performed with aliquots of each DNA preparation. In addition, PCR was performed with an aliquot of Tris buffer (10 mM TrisHCl buffer pH 7.5) containing about 10 copies of the control plasmid which is part of the MYCOTOOL kit (positive control).

Again, the effect that the presence of CHO cell DNA (a) suppresses non-specific PCR amplification products and (b) increases sensitivity of PCR detection could be verified.

### Example 8

### Detection of target sequences in the 16S-rRNA complement of prokaryotic DNA in "inoculated" samples of allantois fluid from embryonated chicken eggs, addition of different concentrations of CHO cell DNA to the PCR reaction mixture

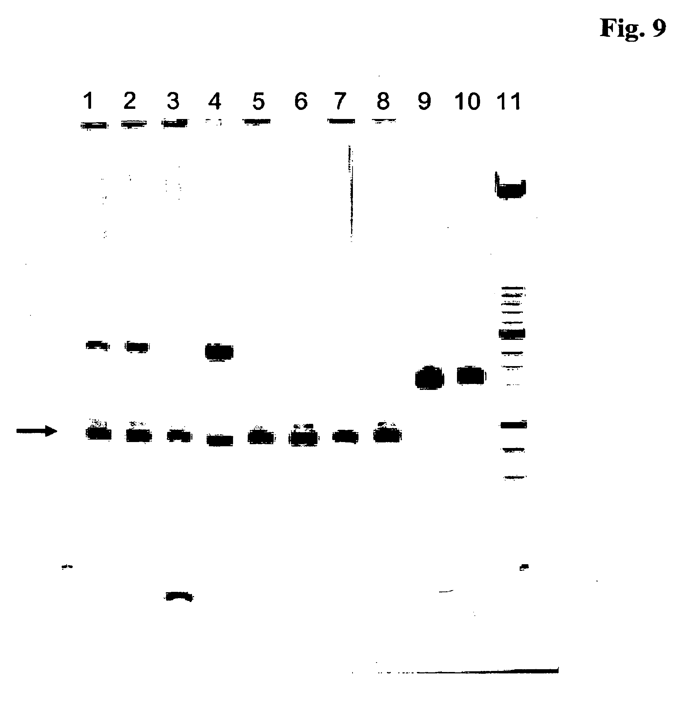

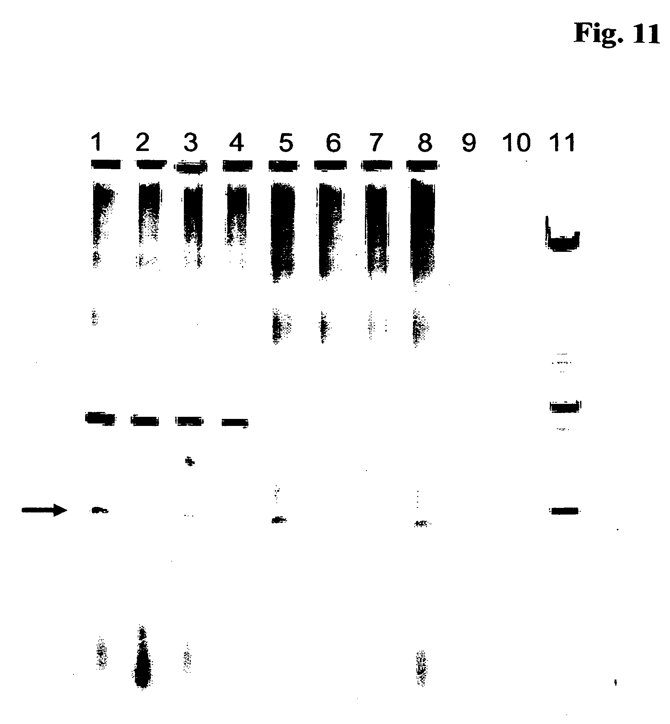

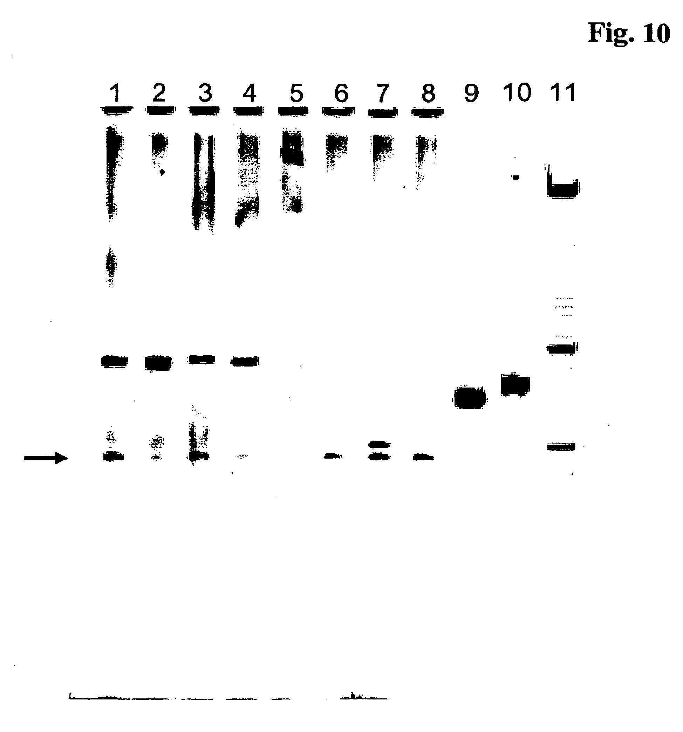

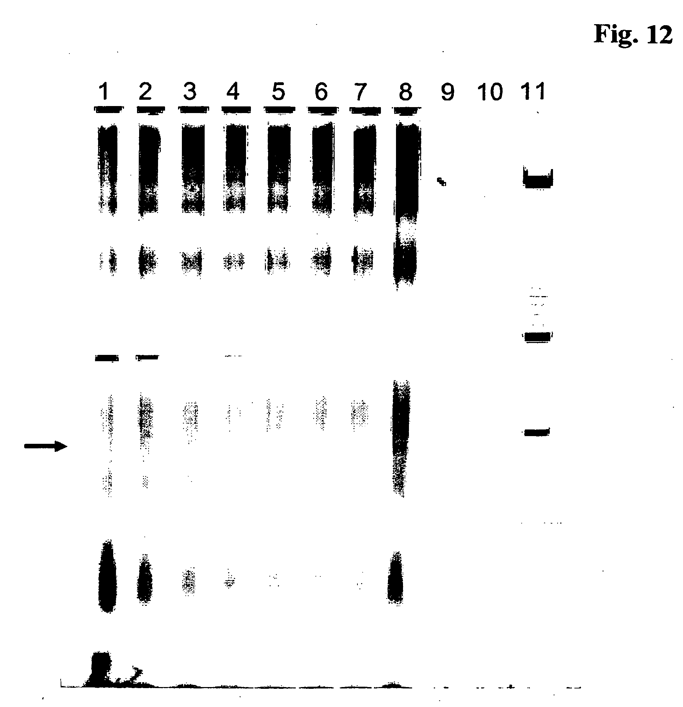

The experiment was conducted as described in Example 7 with the exception that no CHO cell DNA was added prior to DNA purification. Instead, CHO cell DNA was added to the reaction mixture prior to starting PCR. The concentration of CHO cell DNA in the reaction mixture was 0, 2.5, 5, and 10 µg per 50 µl (volume of the PCR reaction mixture). This corresponds to 0 µg/µl, 0.05 µg/µl, 0.1 µg/µl, and 0.2 µg/µl in the final reaction mixture, i.e. the reaction mixture just prior to starting the PCR reaction. Figures 9-12 show the results.

### Example 9

### Detection of target sequences in the 16S-rRNA complement of prokaryotic DNA in spiked aqueous buffer

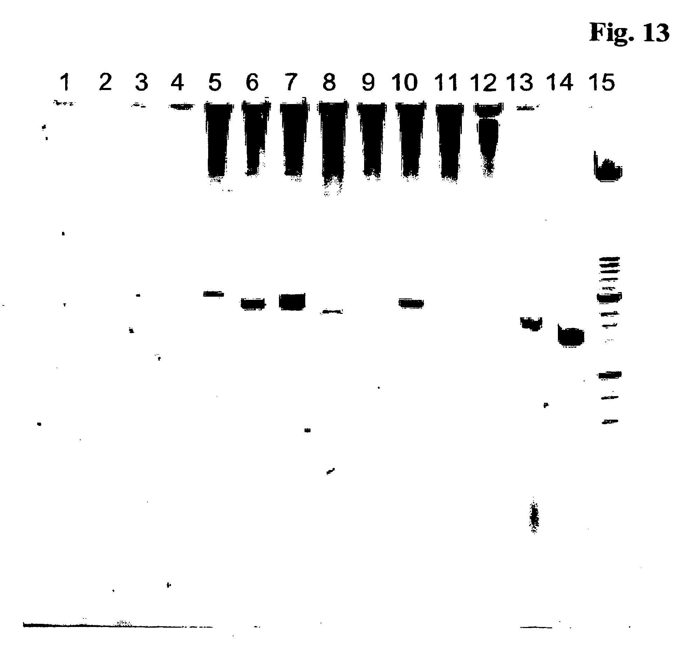

The experiment was conducted as described in Example 5 with the exception that the concentration of A. laidlawii DNA was 3 cfu per 1 ml of buffer and instead of CHO cell DNA calf thymus was added at a concentration of 200 µg per 1 ml of buffer prior to DNA purification. Figure 13 shows the results.

Notably, the PCR fragments generated in the presence of calf thymus DNA show a certain variation in size, unlike the fragments produced in the presence of CHO cell DNA, e.g. those shown in Figure 6 (in particular).

Thus, calf thymus DNA is not suited to suppress the formation of PCR artifacts. In view of these results it was concluded that the ability of CHO cell DNA, rather than calf thymus DNA (and other eukaryotic genomic DNAs), could provide the desired effect due to the fact that the MYCOTOOL PCR based on the primers of SEQ ID NO:1 and SEQ ID NO:2 initially was adapted to CHO cell cultures and culture supernatants. At the time the optimization was made it was not evident that the genomic DNA of the CHO cells provided a "background" which for the primers appears to beneficial in that the "background" apparently suppresses the formation of artifacts. Genomic DNA from other species is different in composition and apparently not able to suppress formation of non-specific artifacts (or less efficient in doing so).

### Example 10

### Detection of target sequences in the 16S-rRNA complement of prokaryotic DNA in "inoculated" samples of allantois fluid from embryonated chicken eggs, addition of calf thymus DNA

The experiment was conducted as described in Example 7 with the exception that instead of CHO cell DNA calf thymus was added prior to DNA purification.

Four different samples were provided: (i) inoculated allantois fluid without calf thymus DNA, (ii) non inoculated allantois fluid without calf thymus DNA, (iii) inoculated allantois fluid with calf thymus DNA added, and (iv) non inoculated allantois fluid with calf thymus DNA added. In the samples of (iii) and (iv) the concentration of the calf thymus DNA was 200 mg per 1 ml of allantois fluid.

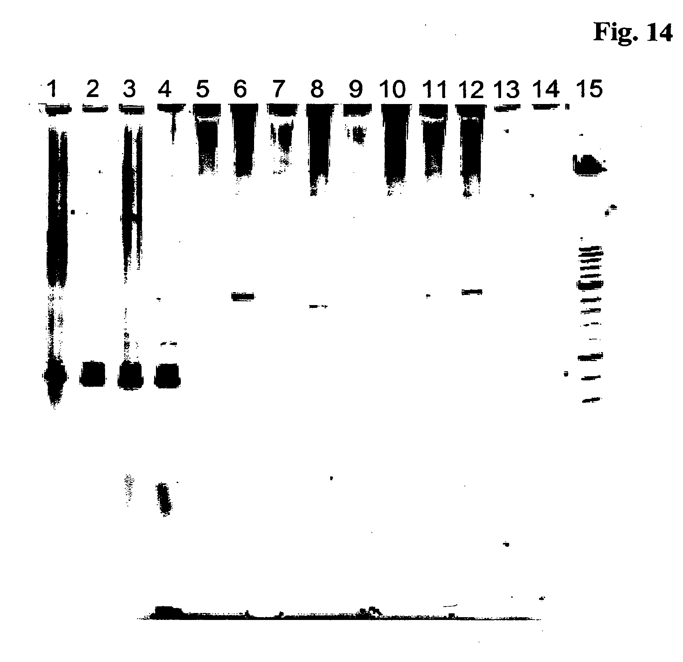

Figure 14 shows the results. Basically, the conclusion is similar as in Example 9. Again, non-specific amplification products were produced, in contrast to PCR in the presence of CHO cell DNA.

## Claims

1. An improved method for determining the presence or absence of a bacterial contaminant in a sample with biological material, the bacterial contaminant being selected from the group consisting of Mycoplasma hyorhinis, Mycoplasma arginini, Mycoplasma pneumoniae, Mycoplasma fermentans, Mycoplasma orale, Mycoplasma pirium, Acholeplasma laidlawii and Spiroplasma mirium, and said method comprising the steps of
(a) processing the sample and purifying nucleic acids from the processed sample; followed by
(b) forming a composition for a PCR-based amplification reaction (reaction mixture), the composition including
- a first primer according to SEQ ID NO:1,
- a second primer according to SEQ ID NO:2, and
- the purified nucleic acids of step (a) or a measured fraction thereof as a template; followed by
(c) performing a polymerase chain reaction (PCR) with the composition of step (b); followed by
(d) detecting the presence or absence of an amplified target sequence, wherein the presence of said amplified target sequence indicates the presence of the bacterial contaminant in the sample, and the absence of said amplified target sequence indicates the absence of the bacterial contaminant in the sample;
the improvement being **characterized in that** relative to the volume of the sample a predetermined amount of DNA from CHO cells is added to (i) the sample, or (ii) the processed sample of step (a), or (iii) the purified nucleic acids obtained in step (a), or (iv) the composition of step (b), whereby the added DNA from CHO cells reduces unspecific amplification in step (d).

2. The method of claim 1, **characterized in that** the predetermined concentration of DNA per ml of the liquid sample is in the range of 10 µg per 1 ml of sample to 250 µg per 1 ml of sample.

3. The method according to any of the claims 1 and 2, **characterized in that** the liquid sample is selected from the group consisting of cell culture medium with cultured cells, cell-free culture supernatant, and amniotic fluid.

4. The method according to claim 3, **characterized in that** the liquid sample is amniotic fluid.

5. The method according to claim 4, **characterized in that** the amniotic fluid is from embryonated eggs.

6. The method according to claim 1, **characterized in that** in step (d) the amplified target sequence is a specific amplification product with a size in the range of about 100 bp to about 1500 bp.

7. The method according to claim 1, **characterized in that** the DNA from CHO cells is free of prokaryotic DNA.

8. A composition comprising allantoic fluid from embryonated chicken eggs and DNA from CHO cells.

9. The composition according to any claim 8, further comprising a lysis reagent selected from a chaotropic agent and a protease.

10. A composition comprising (i) purified nucleic acids from a sample selected from the group consisting of amniotic fluid, a suspension of eukaryotic cells, and a supernatant from a suspension of eukaryotic cells, wherein the eukaryotic cells are derived from a human or an animal species other than Chinese hamster, and (ii) DNA from CHO cells which is free of prokaryotic DNA.

11. The composition according to claim 10, further comprising a first primer according to SEQ ID NO:1, a second primer according to SEQ NO:2, nucleotide triphosphates, and a thermostable DNA polymerase.

12. The composition according to claim 11, further comprising an intercalating dye.

13. A kit comprising in separate containers (i) a lysis reagent, (ii) purified DNA from CHO cells at a predefined concentration, said DNA being free of prokaryotic DNA, and (iii) a first primer according to SEQ ID NO:1 and a second primer according to SEQ ID NO:2.

14. The use of a composition according to any of the claims 10 to 12 for amplifying DNA of a prokaryotic contaminant from DNA isolated from a sample selected from the group consisting of amniotic fluid, a suspension of eukaryotic cells, and a supernatant from a suspension of eukaryotic cells.

## Patentansprüche

1. Verbessertes Verfahren zum Bestimmen der Anwesenheit oder Abwesenheit einer bakteriellen Kontaminante in einer Probe mit biologischem Material, wobei die bakterielle Kontaminante aus der Gruppe bestehend aus Mycoplasma hyorhinis, Mycoplasma arginini, Mycoplasma pneumoniae, Mycoplasma fermentans, Mycoplasma orale, Mycoplasma pirium, Acholeplasma laidlawii und Spiroplasma mirium ausgewählt ist und wobei das Verfahren die folgenden Schritte umfasst:
(a) Verarbeiten der Probe und Aufreinigen der Nukleinsäuren aus der verarbeiteten Probe; und danach
(b) Bilden einer Zusammensetzung für eine Amplifikation auf PCR-Basis (Reaktionsansatz), wobei die Zusammensetzung Folgendes beinhaltet:
- einen ersten Primer gemäß SEQ ID NO: 1,
- einen zweiten Primer gemäß SEQ ID NO: 2, und
- die aufgereinigten Nukleinsäuren aus Schritt (a) oder einen abgemessenen Teil davon als Matrize; und danach
(c) Durchführen einer Polymerasenkettenreaktion (PCR) mit der Zusammensetzung von Schritt (b); und danach
(d) Nachweisen der Anwesenheit oder Abwesenheit einer amplifizierten Zielsequenz, wobei die Anwesenheit der amplifizierten Zielsequenz die Anwesenheit der bakteriellen Kontaminante in der Probe anzeigt und wobei die Abwesenheit der amplifizierten Zielsequenz die Abwesenheit der bakteriellen Kontaminante in der Probe anzeigt;
wobei die Verbesserung **dadurch gekennzeichnet ist, dass** relativ zu dem Volumen der Probe eine vorbestimmte DNA-Menge von CHO-Zellen (i) zu der Probe oder (ii) zu der verarbeiteten Probe von Schritt (a) oder (iii) zu den in Schritt (a) erhaltenen aufgereinigten Nukleinsäuren oder (iv) zu der Zusammensetzung von Schritt (b) hinzugefügt wird, wobei die hinzugefügte CHO-Zellen-DNA die unspezifische Amplifikation in Schritt (d) reduziert.

2. Verfahren nach Anspruch 1, **dadurch gekennzeichnet, dass** die vorbestimmte DNA-Konzentration pro ml der flüssigen Probe im Bereich von 10 µg pro 1 ml Probe bis 250 µg pro 1 ml Probe liegt.

3. Verfahren nach einem der Ansprüche 1 und 2, **dadurch gekennzeichnet, dass** die flüssige Probe aus der Gruppe bestehend aus Zellkulturmedium mit kultivierten Zellen, zellfreiem Kulturüberstand und Amnionflüssigkeit ausgewählt ist.

4. Verfahren nach Anspruch 3, **dadurch gekennzeichnet, dass** es sich bei der flüssigen Probe um Amnionflüssigkeit handelt.

5. Verfahren nach Anspruch 4, **dadurch gekennzeichnet, dass** die Amnionflüssigkeit von angebrüteten Eiern stammt.

6. Verfahren nach Anspruch 1, **dadurch gekennzeichnet, dass** in Schritt (d) die amplifizierte Zielsequenz ein spezifisches Amplifikationsprodukt mit einer Größe im Bereich von ungefähr 100 bp bis ungefähr 1500 bp ist.

7. Verfahren nach Anspruch 1, **dadurch gekennzeichnet, dass** die CHO-Zellen-DNA frei von prokaryontischer DNA ist.

8. Zusammensetzung, die Allantois-Flüssigkeit von angebrüteten Hühnereiern und CHO-Zellen-DNA umfasst.

9. Zusammensetzung nach einem Anspruch 8, die weiterhin ein Lysereagens, ausgewählt aus einem chaotropen Agens und einer Protease, umfasst.

10. Zusammensetzung, die in (i) aufgereinigte Nukleinsäuren von einer Probe, ausgewählt aus der Gruppe bestehend aus Amnionflüssigkeit, einer Eukaryontenzellsuspension und Überstand von einer eukaryontischen Zellsuspension, wobei die Eukaryontenzellen von einem Menschen oder einer Tierart, bei der es sich nicht um den Chinesischen Hamster handelt, stammen, und (ii) CHO-Zellen-DNA, die frei von prokaryontischer DNA ist, umfasst.

11. Zusammensetzung nach Anspruch 10, die weiterhin einen ersten Primer gemäß SEQ ID NO: 1, einen zweiten Primer gemäß SEQ ID NO: 2, Nukleotidtriphosphate und eine hitzestabile DNA-Polymerase umfasst.

12. Zusammensetzung nach Anspruch 11, die weiterhin einen interkalierenden Farbstoff umfasst.

13. Kit, das in separaten Containern Folgendes umfasst: (i) ein Lysereagens, (ii) aufgereinigte CHO-Zellen-DNA in einer vordefinierten Konzentration, wobei die DNA frei von prokaryontischer DNA ist, und (iii) einen ersten Primer gemäß SEQ ID NO: 1 und einen zweiten Primer gemäß SEQ ID NO: 2.

14. Verwendung einer Zusammensetzung nach einem der Ansprüche 10 bis 12 zum Amplifizieren von DNA einer prokaryontischen Kontaminante aus DNA, die aus einer Probe, ausgewählt aus der Gruppe bestehend aus Amnionflüssigkeit, einer Eukaryontenzellsuspension und einem Überstand von einer Eukaryontenzellsuspension, isoliert wurde.

## Revendications

1. Procédé amélioré de détermination de la présence ou de l'absence d'un contaminant bactérien dans un échantillon avec un matériau biologique, le contaminant bactérien étant sélectionné parmi le groupe constitué de *Mycoplasma hyorhinis, Mycoplasma arginini, Mycoplasma pneumoniae, Mycoplasma fermentans, Mycoplasma orale, Mycoplasma pirium, Acholeplasma laidlawii* et *Spiroplasma mirium,* et ledit procédé comprenant les étapes de
(a) traitement de l'échantillon et de purification des acides nucléiques de l'échantillon traité ; suivi de
(b) la formation d'une composition pour une réaction d'amplification basée sur la PCR (mélange réactionnel), la composition incluant
- une première amorce selon SEQ ID n° : 1,
- une seconde amorce selon SEQ ID n° : 2, et
- les acides nucléiques purifiés de l'étape (a) ou une fraction dosée de ceux-ci pour servir de matrice ; suivi de
(c) l'exécution d'une réaction en chaîne de la polymérase (PCR) avec la composition de l'étape (b) ; suivi de
(d) la détection de la présence ou de l'absence d'une séquence cible amplifiée, dans laquelle la présence de ladite séquence cible amplifiée indique la présence du contaminant bactérien dans l'échantillon, et l'absence de ladite séquence cible amplifiée indique l'absence du contaminant bactérien dans l'échantillon ;
l'amélioration étant **caractérisée en ce que** relativement au volume de l'échantillon une quantité prédéterminée d'ADN provenant de cellules CHO est ajoutée (i) à l'échantillon, ou (ii) à l'échantillon traité de l'étape (a), ou (iii) aux acides nucléiques purifiés obtenus dans l'étape (a), ou (iv) à la composition de l'étape (b), moyennant quoi l'ADN ajouté provenant des cellules CHO réduit l'amplification non spécifique dans l'étape (d).

2. Procédé selon la revendication 1, **caractérisé en ce que** la concentration prédéterminée d'ADN par ml de l'échantillon liquide se situe dans la plage de 10 µg par 1 ml d'échantillon à 250 µg par 1 ml d'échantillon.

3. Procédé selon l'une quelconque des revendications 1 et 2, **caractérisé en ce que** l'échantillon liquide est sélectionné parmi le groupe constitué d'un milieu de culture cellulaire avec des cellules cultivées, d'un surnageant de culture exempt de cellules, et du liquide amniotique.

4. Procédé selon la revendication 3, **caractérisé en ce que** l'échantillon liquide est du liquide amniotique.

5. Procédé selon la revendication 4, **caractérisé en ce que** le liquide amniotique provient d'oeufs embryonnaires.

6. Procédé selon la revendication 1, **caractérisé en ce que** dans l'étape (d) la séquence cible amplifiée est un produit d'amplification spécifique avec une taille dans la plage d'environ 100 pb à environ 1 500 pb.

7. Procédé selon la revendication 1, **caractérisé en ce que** l'ADN provenant de cellules CHO est exempt d'ADN procaryote.

8. Composition comprenant du liquide allantoïque provenant d'oeufs embryonnaires de poulet et d'ADN provenant de cellules CHO.

9. Composition selon l'une quelconque des revendications 8, comprenant en outre un réactif de lyse sélectionné parmi un agent chaotropique et une protéase.

10. Composition comprenant (i) des acides nucléiques purifiés provenant d'un échantillon sélectionné parmi le groupe constitué du liquide amniotique, d'une suspension de cellules eucaryotes, et d'un surnageant provenant d'une suspension de cellules eucaryotes, dans laquelle les cellules eucaryotes sont dérivées d'une espèce humaine ou animale autre que le hamster chinois et (ii) d'ADN provenant de cellules CHO qui est exempt d'ADN procaryote.

11. Composition selon la revendication 10, comprenant en outre une première amorce selon SEQ ID n° : 1, une seconde amorce selon SEQ ID n° : 2, des triphosphates nucléotidiques, et une ADN polymérase thermostable.

12. Composition selon la revendication 11, comprenant en outre un marqueur intercalant.

13. Trousse comprenant dans des récipients séparés (i) un réactif de lyse, (ii) de l'ADN purifié provenant de cellules CHO en une concentration prédéfinie, ledit ADN étant exempt d'ADN procaryote, et (iii) une première amorce selon SEQ ID n° : 1 et une seconde amorce selon SEQ ID n° : 2.

14. Utilisation d'une composition selon l'une quelconque des revendications 10 à 12 pour l'amplification d'ADN d'un contaminant procaryote provenant d'ADN isolé d'un échantillon sélectionné parmi le groupe constitué du liquide amniotique, d'une suspension de cellules eucaryotes, et d'un surnageant provenant d'une suspension de cellules eucaryotes.


### SEQUENCE LISTING

<110> Roche Diagnostics GmbH F. Hoffmann-La Roche AG
<120> Improved detection of bacterial (mollicutes) contamination
<130> 26237
<150> EP09009965.6
   <151> 2009-08-01
<160> 4
<170> PatentIn version 3.5
<210> 1
   <211> 21
   <212> DNA
   <213> Artificial Sequence
<220>
   <223> universal primer (forward) for the detection of Mycoplasma and related species
<400> 1
   ggcgaatggg tgagtaacac g 21
<210> 2
   <211> 22
   <212> DNA
   <213> Artificial Sequence
<220>
   <223> universal primer (reverse) for the detection of Mycoplasma and related species
<400> 2
   cggataacgc ttgcgaccta tg 22
<210> 3
   <211> 21
   <212> DNA
   <213> Chinese hamster ovary (CHO) cells
<220>
   <221> misc_feature
   <223> forward primer specific for a target sequence in the Glyceraldehyde 3-phosphate dehydrogenase gene
<400> 3
   caaaggcaca gtcaaggctg a 21
<210> 4
   <211> 21
   <212> DNA
   <213> Chinese hamster ovary (CHO) cells
<220>
   <221> misc feature
   <223> reverse primer specific for a target sequence in the Glyceraldehyde 3-phosphate dehydrogenase gene
<400> 4
   tggtgaagac gccagtagat t 21